# EUROPÄISCHE PATENTSCHRIFT

(11) **EP 2 038 392 B1**
(45) Veröffentlichungstag und Bekanntmachung des Hinweises auf die Patenterteilung: **04.12.2013**
(21) Anmeldenummer: 07786873.5
(22) Anmeldetag: 28.06.2007
(51) Int. Cl.: C11D 3/00, C11D 3/12, A61K 8/29, A61Q 19/00

(54) **DESINFIZIERENDES HAUTBEHANDLUNGSMITTEL**
DISINFECTANT SKIN CARE PRODUCT
PRODUIT DE TRAITEMENT POUR LA PEAU DÉSINFECTANT

(30) Priorität: 07.07.2006 DE 102006031897; 23.04.2007 DE 102007019428; 23.05.2007 DE 102007024377
(43) Veröffentlichungstag der Anmeldung: 25.03.2009
(73) Patentinhaber: Henkel AG & Co. KGaA, 40589 Düsseldorf (DE)
(72) Erfinder: BANOWSKI, Bernhard, 40597 Düsseldorf (DE); MEINE, Georg, 40822 Mettmann (DE); COX, Bruce, 40489 Düsseldorf (DE)
(86) Internationale Anmeldenummer: PCT/EP2007/056457
(87) Internationale Veröffentlichungsnummer: WO 2008/003632

(56) Entgegenhaltungen:
- WO-A-01/41727
- WO-A-02/50224
- WO-A-02/083830
- WO-A-03/002703
- WO-A-03/061707
- WO-A-2005/108505
- DE-A1-102006 037 632
- GB-A- 2 358 638
- JP-A- 2000 136 112
- KR-A- 20040 101 880
- US-A1- 2005 249 760
- US-A1- 2007 031 461

## Beschreibung

Die Erfindung betrifft ein antiseptisches kosmetisches Hautreinigungs- und/oder -pflegemittel auf der Basis eines lichtaktiven Metalloxids sowie die kosmetische Verwendung des Mittels zur Prophylaxe, Verminderung, Beseitigung und/oder Linderung von bakteriellen, fungiziden und/oder viralen Infektionen.

Die Haut ist mit einer Fläche von bis zu 2 m² das größte Körperorgan des Menschen und vielfältigen, schädlichen Umwelteinflüssen und Keimen ausgesetzt. Die Zahl der Menschen mit empfindlicher Haut und dermatologischen Problemen, die von leichten Hautirritationen über Sensibilisierungen bis hin zu Allergien reichen können, nimmt stetig zu.

Des Weiteren können durch Hautkontakt Keime übertragen werden, die zu bakteriellen, fungiziden und/oder viralen Infektionen der Haut, der Atemwege oder des Magen-Darm-Trakts führen können. Dementsprechend steigt der Wunsch der Menschen nach kosmetischen oder dermatologischen Zubereitungen, die solchen Problemen vorbeugen oder ihnen abhelfen.

Beispielsweise in öffentlichen sanitären Einrichtungen, in Krankenhäusern oder Schwimmbädern mit mangelhaften Hygienebedingungen ist die Haut besonders gefährdet, mit pathogenen Mikroorganismen befallen zu werden, aus denen schwerwiegende Infektionen entstehen können. Üblicherweise werden als Desinfektionsmittel Aldehyde, quaternäre Ammoniumverbindungen und/oder Phenole eingesetzt. Diese Stoffe können allerdings auf der menschlichen Haut, insbesondere bei häufiger Verwendung in höheren Dosen, Kontaktallergien und Überempfindlichkeitsreaktionen hervorrufen.

Daher besteht der Bedarf nach nicht toxischen antiseptischen Mitteln mit niedrigem Allergiepotential, die sich optimalerweise sowohl in ein Rinse-off- als auch in ein Leave-on-Produkt für die Haut einarbeiten lassen und die Haut effektiv und langanhaltend vor Keimen schützen. Insbesondere erstrebenswert ist ein solcher antiseptischer Wirkstoff, der aus einem solchen Produkt auf die Haut aufzieht und dort reaktiviert werden kann, damit ein permanenter Schutz der Haut vor Keimen erreicht werden kann, ohne die Haut durch ständiges Nachbehandeln mit den Mitteln zu reizen oder übermäßig zu beanspruchen.

In dem Dokument KR 1020040101880A wird eine sterilisierende, die Kopfhaut schützende Seife offenbart, die TiO₂ als Photokatalysator und Germanium enthält. Diese Seifen konnten jedoch nicht alle zuvor genannten Bedürfnisse in zufrieden stellender Weise befriedigen.

Vollkommen überraschend wurde gefunden, dass lichtaktive, modifizierte Metalloxide sich als antiseptische Wirkstoffe für den Einsatz in kosmetischen Hautreinigungs- und -pflegemitten eignen.

Stimuliert durch UV-Lichteinfluss, beispielsweise aus dem Sonnenlicht, bildet das modifizierte Metalloxid aktive Radikale aus, die diejenigen Mikroorganismen, welche für die vorgenannte Infektionen verantwortlich sein können, abtötet, und damit den Hautbefall mit pathogenen Mikroorganismen verhindert oder vermindert.

Gegenstand der Erfindung sind somit antiseptische kosmetische Hautreinigungs- und/oder - pflegemittel, die einen lichtaktiven Wirkstoff auf der Basis eines mit Kohlenstoff modifizierten Metalloxids enthalten.

Die erfindungsgemäßen Metalloxide lassen sich in alle gängigen Hautbehandlungsmittel einarbeiten. Ein besonderer Vorteil dieser Wirkstoffe besteht darin, dass sie aus den Hautbehandlungsmitteln auf die Haut aufziehen und durch Lichteinfluss immer wieder reaktiviert werden können, wodurch eine Langzeitwirkung und damit ein permanenter Schutz vor Keimen und Viren erzielt werden kann.

Der Kohlenstoffgehalt in dem mit Kohlenstoff modifizierten Metalloxid beträgt bevorzugt 0,01 bis 10 Gew.%, mehr bevorzugt 0,05 bis 5 Gew.%, besonders bevorzugt 0,3 bis 1,5 Gew.% und insbesondere 0,4 bis 0,8 Gew.-%.

Die Lichtaktivität des lichtaktiven Wirkstoffs bezieht sich vorteilhafterweise auf natürliches oder künstliches Licht mit einer Wellenlänge im Bereich von 300 bis 1200 nm, vorzugsweise zwischen 380 und 800 nm.

Vorteilhafterweise reicht sogar das Licht, welches durch Glasfenster in geschlossene Wohnräume fällt (diffuses Tageslicht), oder Licht aus technischen Lichtquellen, wie z.B. aus handelsüblichen Glühlampen (Glühbirnen), Halogenlampen, Leuchtstoffröhren, Kompaktleuchtstofflampen (Energiesparlampen) sowie aus Lichtquellen auf Basis von Leuchtdioden aus, um die gewünschte Radikalbildung und damit den Schutz vor pathogenen Keimen und Viren bei der kosmetischen Behandlung der Haut in Gang zu setzten.

Die spezifische Oberfläche der erfindungsgemäß geeigneten modifizierten Metalloxide beträgt nach BET 200 bis 350 m²/g und die Teilchengröße liegt bevorzugt im Bereich von 2 bis 600 nm, besonders bevorzugt im Bereich von 200 bis 400 nm.

Das modifizierte Metalloxid wird in den erfindungsgemäßen Mitteln - bezogen auf das gesamte Mittel - üblicherweise in einer Menge von 0,01 bis 20 Gew.%, bevorzugt von 0,05 bis 10 Gew.-% und insbesondere in einer Menge von 0,1 bis 5 Gew.% eingesetzt.

Bei den lichtaktiven Metalloxiden handelt es sich erfindungsgemäß bevorzugt um Zinkoxid und/oder Titanoxid, insbesondere bevorzugt um Titanoxid.

Ein erfindungsgemäß besonders geeignetes modifiziertes Titandioxid ist unter der Handelsbezeichnung Kronos^{®} vlp 7000 von der Firma Kronos im Handel erhältlich.

Die erfindungsgemäßen Metalloxide lassen sich sowohl in Rinse-off-Produkte wie eine flüssige oder feste Handwaschseife, ein Flüssighandwaschmittel, eine Seife, ein Syndet, ein Duschgel oder ein Duschbad, sowie in Leave-on-Produkte wie eine wässrige, alkoholische oder wässrig/alkoholische Dispersion, eine Öldispersion, eine Salbe, eine Creme, eine Emulsion, eine Suspension, eine Milch, eine Paste, ein Gel, einen Schaum oder ein Spray einarbeiten. Des weiteren ist auch der Einsatz eines Substrats, das mit einem der oben genannten, geeigneten Mittel imprägniert, getränkt oder beschichtet wurde, erfindungsgemäß bevorzugt. Alle diese Ausführungsformen eignen sich in hervorragender Weise zur Behandlung der menschlichen Haut.

Zur Verstärkung der antiseptischen Wirkung der erfindungsgemäßen Hautreinigungs- und/oder - pflegemittel enthalten diese in einer besonders bevorzugten Ausführungsform der Erfindung zusätzlich zu dem lichtaktiven Metalloxid 0,001 bis 5 Gew.-%, bevorzugt 0,005 bis 3 Gew.-% und insbesondere 0,01 bis 1,5 Gew.-% eines oder mehrerer weiterer antibakterieller Wirkstoffe. Diese sind beispielsweise ausgewählt aus Alkoholen wie Ethanol, Propylenglycol, Isopropanol oder Butanol, Glycerinderivaten, Phenolen, halogenierten Phenolen wie Dichlorophen, Bromchlorophen, Chlorcresol, Chlorxylenol, Chlorthymol oder Phenylphenol, etherischen Ölen wie Teebaumöl, Cineol, Eugenol, Geraniol, Limonen, Menthol, Thymol, Hanföl, Borretschöl, Nachtkerzenöl, das Kernöl der schwarzen Johannisbeere, Echiumöl, Trichodesmaöl oder Schwarzkümmelöl, PHB-estern wie die Methyl-, Ethyl, Propyl- oder Butylestern, Sorbinsäure, Benzoesäure, Salicylsäure und/oder einem physiologisch verträglichen Salz oder Ester dieser Säuren, Undecensäurederivaten, Formaldehyd, anorganischen Sulfiten und Bisulfiten, 4-Hydroxybenzoesäure und/oder deren physiologisch verträglichen Salzen oder Estern, Chlorbutanol, Dehydracetsäure, Bronidox, Bronopol, Triclosan, Triclocarben, Chlorcresol, Chlorxylenol, Chlorhexidin, Germall, Dowicil, Baypival und/oder Kathon. Für den Fall, dass Alkohole eingesetzt werden, so kann der Gehalt der Alkohole in den Mitteln bis zu 75 Gew.-% betragen, bezogen auf das Gesamtgewicht des Mittels.

Ganz besonders bevorzugte weitere antibakterielle Wirkstoffe sind quaternäre Ammoniumverbindungen, Ethanol, Propylenglycol, Isopropanol, Butanol, Salicylsäure, Triclosan, Benzylalkohol, Ethylhexylglycerin (beispielsweise das unter dem Handelsnamen Sensiva^{®} SC 50 von der Firma Schülke & Mayr vertriebene Handelprodukt) Triclocarben und/oder Chlorhexidin.

Das Gewichtsverhältnis zwischen dem lichtaktiven Metalloxid und dem weiteren antibakteriellen Wirkstoff beträgt vorzugsweise 200.1 bis 1:10, bevorzugt 100:1 bis 1:5 und insbesondere 50:1 bis 1:1.

Weiterhin erfindungsgemäß bevorzugt sind Hautreinigungs- und/oder -pflegemittel, die weiterhin mindestens ein Verdickungssystem, das die Sedimentation der Metalloxid-Partikel verhindert, enthalten.

Häufig verwendete Verdickungssysteme enthalten Polymere.

Im Folgenden werden einige Beispiele typischer polymerer Verdicker für wässrige Systeme aufgeführt:
Acrylamides Copolymer, Acrylamide/Sodium Acrylate Copolymer, Acrylamide/Sodium Acryloyldimethyltaurate Copolymer, AcrylateslAcetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, AcrylatesNinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/lsophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene/Sodium Maleate Copolymer, Lithium Magnesium Silicate, Lithium Magnesium Sodium Silicate, Sodium Magnesium Silicate, Lithium Magnesium Sodium Fluoride Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium AcrylateNinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Eine weitere Möglichkeit zur Steigerung der Viskosität von kosmetischen Mitteln ist die Verdickung der nicht-wässrigen Phase, der Lipidphase der kosmetischen Mittel. Hierzu werden Polymere eingesetzt, welche nicht wasserlöslich aber kompatibel mit Lipiden sind. Sie werden auch zur Gelbildung von kosmetischen Mitteln mit hohen Lipidanteilen verwendet.

Im Folgenden werden einige dieser Polymere aufgelistet:
Acrylates/C10-30 Alkyl Acrylate Crosspolymer, Adipic Acid/PPG-10 Copolymer, Allyl Methacrylates Crosspolymer, Alumina Magnesium Metasilicate, Aluminum Starch Octenylsuccinate, Beeswax, Behenyl Methacrylate/Perfluorooctylethyl Methacrylate Copolymer, Bispolyethylene Dimethicone, Butadiene/Acrylonitrile Copolymer, Butylene/Ethylene Copolymer, Butylene/Ethylene/Styrene Copolymer, Butylene Glycol Montanate, Butyrospermum Parkii (Shea Butter), C29-70 Acid, C23-43 Acid Pentaerythritol Tetraester, C20-24 Alkyl Dimethicone, C24-28 Alkyl Dimethicone, C1-5 Alkyl Galactomannan, C18-38 Alkyl Hydroxystearoyl Stearate, C20-24 Alkyl Methicone, C24-28 Alkyl Methicone, C30-45 Alkyl Methicone, Candelilla Wax Hydrocarbons, C10-30 Cholesterol/Lanosterol Esters, Cellobiose Octanonanoate, Ceresin, Cerotic Acid, Cetearyl DimethiconeNinyl Dimethicone Crosspolymer, Chlorinated Paraffin, Cholesterol, Cholesteryl Acetate, Cholesteryl Hydroxystearate, Cholesteryl Isostearate, Cholesteryl Macadamiate, Cholesteryl Stearate, C10-40 Hydroxyalkyl Acid Cholesterol Esters, C10-40 Isoalkyl Acid Cholesterol Esters, C10-40 Isoalkyl Acid Octyldodecanol Esters, C10-40 Isoalkyl Acid Phytosterol Esters, C10-40 Isoalkyl Acid Triglyceride, C30-38 Olefin/Isopropyl Maleate/MA Copolymer, Copal, Corn Starch Modified, C6-14 Perfluoroalkylethyl Acrylate/HEMA Copolymer, C6-14 Polyolefin, Decene/Butene Copolymer, Dihydrogenated Tallow Benzylmonium Hectorite, Dilinoleic Acid/Ethylenediamine Copolymer, Dilinoleic Acid/Sebacic Acid/Piperazine/Ethylenediamine Copolymer, Dimethicone Crosspolymer, Dimethicone/Phenyl Vinyl Dimethicone Crosspolymer, DimethiconeNinyl Dimethicone Crosspolymer, Dimethicone/Vinyltrimethylsiloxysilicate Crosspolymer, Diphenyl DimethiconeNinyl Diphenyl Dimethicone/Silsesquioxane Crosspolymer, Divinyldimethicone/Dimethicone Crosspolymer, Dodecanedioic Acid/Cetearyl Alcohol/Glycol Copolymer, Ethylcellulose, Ethylene/Acrylic Acid Copolymer, Ethylene/Acrylic Acid/VA Copolymer, Ethylenediamine/Dimer Tallate Copolymer Bis-Hydrogenated Tallow Amide, Ethylenediamine/Stearyl Dimer Dilinoleate Copolymer, Ethylenediamine/Stearyl Dimer Tallate Copolymer, Ethylene/Octene Copolymer, Ethylene/Propylene Copolymer, Ethylene/Propylene/Styrene Copolymer, Euphorbia Cerifera (Candelilla) Wax, Hydrogenated Butylene/Ethylene/Styrene Copolymer, Hydrogenated Ethylene/Propylene/Styrene Copolymer, Hydrogenated Japan Wax, Hydrogenated Polyisobutene, Hydrogenated Styrene/Butadiene Copolymer, Hydrogenated Styrene/Methyl Styrene/Indene Copolymer, Hydroxypropylcellulose, Isobutylene/Isoprene Copolymer, Lithium Oxidized Polyethylene, Methoxy PEG-17/Dodecyl Glycol Copolymer, Methoxy PEG-22/Dodecyl Glycol Copolymer, Methyl Methacrylate Crosspolymer, Methylstyrene/Vinyltoluene Copolymer, Microcrystalline Wax, Montan Acid Wax, Montan Wax, Myrica Cerifera (Bayberry) Fruit Wax, Nylon-611/Dimethicone Copolymer, Octadecene/MA Copolymer, Oleic/Linoleic/Linolenic Polyglycerides, Ouricury Wax, Oxidized Beeswax, Oxidized Microcrystalline Wax, Oxidized Polyethylene, Oxidized Polypropylene, Ozokerite, Paraffin, PEG-18 Castor Oil Dioleate, PEG-10 Dimethicone Crosspolymer, PEG-12 Dimethicone Crosspolymer, Hydrogenated Castor Oil, PEG-5 Hydrogenated Castor Oil Isostearate, PEG-10 Hydrogenated Castor Oil Isostearate, PEG-20 Hydrogenated Castor Oil Isostearate, PEG-30 Hydrogenated Castor Oil Isostearate, PEG-40 Hydrogenated Castor Oil Isostearate, PEG-50 Hydrogenated Castor Oil Isostearate, PEG-58 Hydrogenated Castor Oil Isostearate, PEG-50 Hydrogenated Castor Oil Succinate, PEG-5 Hydrogenated Castor Oil Triisostearate, PEG-10 Hydrogenated Castor Oil Triisostearate, PEG-15 Hydrogenated Castor Oil Triisostearate, PEG-20 Hydrogenated Castor Oil Triisostearate, PEG-30 Hydrogenated Castor Oil Triisostearate, PEG-40 Hydrogenated Castor Oil Triisostearate, PEG-60 Hydrogenated Castor Oil Triisostearate, PEG-5 Lanolinamide, PEG-5 Oleamide Dioleate, Phthalic Anhydride/Butyl Benzoic Acid/Propylene Glycol Copolymer, Phthalic Anhydride/Glycerin/Glycidyl Decanoate Copolymer, Phthalic Anhydride/Trimellitic Anhydride/Glycols Copolymer, Piperylene/Butene/Pentene Copolymer, Polybutene, Polybutylene Terephthalate, Polycyclopentadiene, Polydipentene, Polyethylene, Polyethylene Terephthalate, Polyglyceryl-3 Polyricinoleate, Polyglyceryl-4 Polyricinoleate, Polyglyceryl-5 Polyricinoleate, Polyglyceryl-10 Polyricinoleate, Polyisobutene, Polyisoprene, Polypentene, Polyperfluoroethoxymethoxy Difluoromethyl Distearamide, Polypropylene, Polysilicone-4, Polysilicone-5, Polysilicone-17, Polystyrene, Polyvinyl Butyral, Polyvinyl Laurate, Potassium Oxidized Microcrystalline Wax, Potassium PEG-50 Hydrogenated Castor Oil Succinate, PVM/MA Decadiene Crosspolymer, PVP/Decene Copolymer, Rhus Succedanea Fruit Wax, Rosin, Silica Dimethicone Silylate, Silica Dimethyl Silylate, Simmondsia Chinensis (Jojoba) Seed Wax, Sodium PVM/MA/Decadiene Crosspolymer, Spent Grain Wax, Steareth-10 Allyl Ether/Acrylates Copolymer, Steareth-60 Cetyl Ether, Stearoxymethicone/Dimethicone Copolymer, Stearyl Methacrylate/Perfluorooctylethyl Methacrylate Copolymer, Styrene/Methacrylamide/Acrylates Copolymer, Synthetic Beeswax, Synthetic Candelilla Wax, Synthetic Carnauba, Synthetic Japan Wax, Synthetic Wax, TDI Oxidized Microcrystalline Wax, Tricontanyl PVP, Trifluoropropyl Dimethicone Crosspolymer, Trifluoropropyl Dimethicone/Trifluoropropyl Divinyldimethicone Crosspolymer, Trifluoropropyl Dimethicone/Vinyl Trifluoropropyl Dimethicone/Silsesquioxane Crosspolymer, Trimethylpentanediol/Isophthalic Acid/Trimellitic Anhydride Copolymer, Trimethylsiloxysilicate/Dimethiconol Crosspolymer, Vinyl Dimethicone/Lauryl Dimethicone Crosspolymer, Vinyl Dimethicone/Methicone Silsesquioxane Crosspolymer, VP/Eicosene Copolymer, VP/Hexadecene Copolymer.

Selbstverständlich zählen auch die emulsionsstabilisierenden Polymere zu den erfindungsgemäß bevorzugten Polymeren. Hierunter sind Polymere zu verstehen, welche den Aufbau und die Stabilisierung von Emulsionen (O/W und W/O sowie multiple Emulsionen) wesentlich unterstützen. Tenside und Emulgatoren sind selbstverständlich die wesentlichen Bestandteile, jedoch tragen die stabilisierenden Polymere durch eine positive Beeinflussung der kontinuierlichen oder der dispersen Phase zu einer Verringerung der Koaleszenz der emulgierten Tröpfchen bei. Diese positive Beeinflussung kann auf einer elektrischen Abstoßung, einer Erhöhung der Viskosität oder einer Filmbildung auf der Tröpfchenoberfläche beruhen. Diese Eigenschaften der betreffenden Polymere können auch in den erfindungsgemäßen Zusammensetzungen besonders vorteilhaft verwendet werden, um die erfindungsgemäßen Wirkstoffe, die modifizierten Titandioxidpartikel, stabil in den erfindungsgemäßen Zusammensetzungen dispergiert zu halten.

Beispiele für derartige Polymere sind Acrylamide/Sodium Acryloyldimethyltaurate Copolymer, Acrylates/Aminoacrylates/C10-30 Alkyl PEG-20 Itaconate Copolymer, Acrylates/C10-30 Alkyl Acrylate Crosspolymer, Acrylates/Stearyl Methacrylate Copolymer, Acrylates/Vinyl Isodecanoate Crosspolymer, Alcaligenes Polysaccharides, Allyl Methacrylates Crosspolymer, Ammonium Acryloyldimethyltaurate/Beheneth-25 Methacrylate Crosspolymer, Ammonium AcryloyldimethyltaurateNinyl Formamide Copolymer, Ammonium Alginate, Ammonium Phosphatidyl Rapeseedate, Ammonium Polyacrylate, Ammonium Polyacryloyldimethyl Taurate, Ammonium Shellacate, Arachidyl Alcohol, Astragalus Gummifer Gum, Beeswax, Bentonite, Calcium Carboxymethyl Cellulose, Calcium Carrageenan, Calcium Potassium Carbomer, Calcium Starch Octenylsuccinate, C1-5 Alkyl Galactomannan, C18-38 Alkyl Hydroxystearoyl Stearate, Carbomer, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Chitosan Lauroyl Glycinate, Cholesterol, Cholesterol/HDI/Pullulan Copolymer, Corn Starch/Acrylamide/Sodium Acrylate Copolymer, C12-14 Sec-Pareth-3, C12-14 Sec-Pareth-5, C12-14 Sec-Pareth-7, C12-14 Sec-Pareth-8, C12-14 Sec-Pareth-9, C12-14 Sec-Pareth-12, C12-14 Sec-Pareth-15, C12-14 Sec-Pareth-20, C12-14 Sec-Pareth-30, C12-14 Sec-Pareth-40, C12-14 Sec-Pareth-50, Cyamopsis Tetragonoloba (Guar) Gum, Dimethicone Crosspolymer, Dimethicone Crosspolymer-2, Dimethicone Ethoxy Glucoside, Euphorbia Cerifera (Candelilla) Wax, Gellan Gum, Hydrolyzed Beeswax, Hydrolyzed Candelilla Wax, Hydrolyzed Carnauba Wax, Hydrolyzed Collagen PG-Propyl Dimethiconol, Hydrolyzed Sunflower Seed Wax, Hydroxybutyl Methylcellulose, Hydroxyethyl Acrylate/Sodium Acryloyldimethyl Taurate Copolymer, Hydroxyethylcellulose, Hydroxyethyl Ethylcellulose, Hydroxyethyl Isostearyloxy Isopropanolamine, Hydroxypropylcellulose, Hydroxypropyl Cyclodextrin, Hydroxypropyl Guar, Hydroxypropyl Methylcellulose, Hydroxypropyl Xanthan Gum, Isopropyl Ester of PVM/MA Copolymer, Lanolin, Lanolin Alcohol, Magnesium Alginate, Maltodextrin, Methoxy PEG-17/Dodecyl Glycol Copolymer, Methoxy PEG-22/Dodecyl Glycol Copolymer, Methylcellulose, Methyl Hydroxyethylcellulose, Microcrystalline Cellulose, Microcrystalline Wax, Montmorillonite, Moroccan Lava Clay, Myrica Cerifera (Bayberry) Fruit Wax, Octadecene/MA Copolymer, Oleic/Linoleic/Linolenic Polyglycerides, Ozokerite, Pectin, PEG-350, PEG-400, PEG-500, PEG-12 Carnauba, PEG-12 Dimethicone Crosspolymer, PEG-22/Dodecyl Glycol Copolymer, PEG-45/Dodecyl Glycol Copolymer, PEG-6 Hydrogenated Palmamide, PEG-100/IPDI Copolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG/PPG-20/23 Dimethicone, PEG/PPG-23/6 Dimethicone, PEG/PPG-8/3 Laurate, PEG/PPG-10/3 Oleyl Ether Dimethicone, Polyacrylic Acid, Polyethylene, Polyethylene/Isopropyl Maleate/MA Copolyol, Polyglyceryl-2 Diisostearate/IPDI Copolymer, Polypropylene Terephthalate, Polysilicone-16, Polyvinyl Acetate, Potassium Alginate, Potassium Carbomer, Potassium, Carrageenan, Potassium Dextrin Octenylsuccinate, Potassium Polyacrylate, Potassium Undecylenoyl Alginate, Potassium Undecylenoyl Carrageenan, Potassium Undecylenoyl Hydrolyzed Corn Protein, Potassium Undecylenoyl Hydrolyzed Soy Protein, Potassium Undecylenoyl Hydrolyzed Wheat Protein, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-2 Tocophereth-5, PPG-5 Tocophereth-2, PPG-10 Tocophereth-30, PPG-20 Tocophereth-50, PVM/MA Copolymer, PVP, PVP/Decene Copolymer, PVP Montmorillonite, Pyrus Malus (Apple) Fiber, Saccharated Lime, Sclerotium Gum, Sodium Acrylate/Acryloyldimethyl Taurate Copolymer, Sodium Acrylates/Vinyl Isodecanoate Crosspolymer, Sodium AcrylateNinyl Alcohol Copolymer, Sodium Carbomer, Sodium Carboxymethyl Dextran, Sodium Carboxymethyl Starch, Sodium Carrageenan, Sodium Cellulose Sulfate, Sodium C4-12 Olefin/Maleic Acid Copolymer, Sodium Cyclodextrin Sulfate, Sodium Dextrin Octenylsuccinate, Sodium Polyacrylate, Sodium Polyacrylate Starch, Sodium Polyacryloyldimethyl Taurate, Sodium Polymethacrylate, Sodium Polynaphthalenesulfonate, Sodium Polystyrene Sulfonate, Sodium Starch Octenylsuccinate, Sodium/TEA-Undecylenoyl Alginate, Sodium/TEA-Undecylenoyl Carrageenan, Sodium Tocopheryl Phosphate, Starch Hydroxypropyltrimonium Chloride, Stearylvinyl Ether/MA Copolymer, Sterculia Urens Gum, Styrene/MA Copolymer, Sucrose Polypalmate, Synthetic Beeswax, Synthetic Wax, Tamarindus Indica Seed Gum, TEA-Alginate, TEA-Dextrin Octenylsuccinate, Undecylenoyl Inulin, Undecylenoyl Xanthan Gum, Welan Gum, Xanthan Gum, Zinc Undecylenoyl Hydrolyzed Wheat Protein.

Besonders bevorzugte verdickende und/oder suspendierend wirkende Polymere sind Acrylamides Copolymer, Acrylamide/Sodium Acrylate Copolymer, Acrylamide/Sodium Acryloyldimethyltaurate Copolymer, Acrylates/Beheneth-25 Methacrylate Copolymer, Acrylates/C10-30 Alkyl Acrylate Crosspolymer, Acrylates/Ceteth-20 Itaconate Copolymer, Chitosan Carbomer, Guar Hydroxypropyltrimonium Chloride, Hectorite, Bentonite, Laponit, Hydrated Silica, Hydrogenated Potato Starch, Hydroxypropyl Starch, Hydroxypropyl Starch Phosphate, Hydroxypropyl Xanthan Gum, bzw. die Handelsprodukte mit den Verkaufsbezeichnungen Carbopol^{®}, Pemulen^{®}, Jaguar^{®}, Acrysol^{®} oder Salcare^{®}.

Die Polymere, welche verdickend und/oder suspendierend wirken, sind in den erfindungsgemäßen Zusammensetzungen bevorzugt in Mengen von 0,01 bis 30 Gew.%, bezogen auf die gesamte Zusammensetzung, enthalten. Mengen von 0,01 bis 25, insbesondere von 0,01 bis 15 Gew.%, sind besonders bevorzugt. Höchst bevorzugt sind Mengen von 0,05 Gew.% bis zu 5,0 Gew.% jeweils bezogen auf die gesamte Zusammensetzung.

Die gewünschte Verdickungswirkung der erfindungsgemäßen Zubereitungen kann auch durch den Zusatz von Elektrolyten erzielt bzw. verstärkt werden. Erfindungsgemäß geeignete Elektrolyte enthalten eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektroiyte können vorteilhaft verwendet werden, beispielsweise Lactate, Acetate, Benzoate, Propio- nate, Tartrate, Citrate und andere mehr. Vergleichbare Effekte sind auch durch Ethylendi- amintetraessigsaeure und deren Salze zu erzielen. Als Kationen der Salze werden bevorzugt Ammonium,- Alkylammonium,-Alkalimetall-, Erdalkalimetall,- Magnesium-, Eisen- bzw. Zinkionen verwendet. Es bedarf an sich keiner Erwähnung, dass in Kosmetika nur physiologisch unbedenkliche Elektrolyte verwendet wer- den sollten. Besonders bevorzugte Elektrolyte sind Kaliumchlorid, Natriumchlorid, Magnesiumsulfat, Zinksulfat und Mischungen daraus. Ebenfalls vorteilhaft sind Salzmischungen wie sie im natuerlichen Salz vom Toten Meer auftreten.

Die Konzentration des oder der Elektrolyte sollte etwa 0,1 - 10,0 Gew.%, besonders vor- teilhaft etwa 0,3 - 8,0 Gew.% betragen, bezogen auf das Gesamtgewicht der Zubereitung.

In einer weiteren bevorzugten Ausführungsform der Erfindung können zur Verdickung der erfindungsgemäßen Zubereitungen auch Polymere mit verdickender Wirkung verwendet, die man als Assoziativverdicker bezeichnet. Dies können beispielsweise (müssen jedoch nicht ausschließich) Polymere auf Acrylatbasis sein, die z. T. relativ hohe Molekularmassen im Bereich von > 1.000.000 g/Mol aufweisen können. Sie werden in Mengen von 0,01 bis 10 Gew.%, beispielsweise in Mengen von 0,5 bis 4 Gew.%, in den waschaktiven, spülaktiven oder reinigungsaktiven Formulierungen verwendet. Geeignete Produkte sind beispielsweise die von der Firma Rohm & Haas unter der Bezeichnung Acusol^{R} vertriebenen Produkte, die überwiegend Polyacrylate mit unterschiedlicher Alkylierung oder Vernetzung oder hydrophob modifizierte nichtionische Polyole sind, oder die von der Firma B.F. Goodrich unter der Bezeichnung Carbopol^{R} vertriebenen Produkte, die Polyacrylate oder Copolymere aus Acrylsäure und alkylierten (vorzugsweise C₅- bis C₁₀-alkylierten) Acrylsäuren sind, oder die von der Firma B.F. Goodrich unter der Bezeichnung Pemulen^{R} vertriebenen Produkte, die hochmolekulare Acrylsäure-Copolymere vom Typ Acrylat/C₁₀- bis C₃₀-Alkylacrylat-Crosspolymer sind. Neben den vorgenannten Assoziativ-Verdickern sind auch Polyacrylat- bzw. Polyurethan-Verdicker verwendbar, die über einen anderen Wirkmechanismus (also nicht assoziativ) bei Anwesenheit kleiner Mengen Wasser die Möglichkeit zur Einstellung der Viskosität bieten.

In einer ersten bevorzugten Ausführungsform der Erfindung handelt es sich bei dem erfindungsgemäßen Hautreinigungs- und/oder -pflegemittel um ein Rinse-off-Produkt wie eine flüssige oder feste Handwaschseife, ein Flüssighandwaschmittel, eine Seife, ein Syndet, ein Duschgel oder ein Duschbad, das neben den vorgenannten Bestandteilen mindestens einen Vertreter aus der Gruppe der Tenside (die anionisch, amphoter, zwitterionisch, nichtionisch, kationisch sein können), der Seifen, der kationischen Polymere, der wasserunlöslichen Ölkomponenten, der Vitamine, der Provitamine, der Proteinhydrolysate, der Pflanzenextrakte, der Aminosäuren, der wasserunlöslichen Silikone, der wasserlöslichen Silikone, der Trübungsmittel und/oder der Perlglanzmittel enthält.

Für den Fall, dass es sich bei der erfindungsgemäßen Zubereitung um eine Seife oder ein Syndet handelt, so sind diese bevorzugt analog den Zubereitungen des Dokuments WO 2007/025645 A1 zusammengesetzt. Daneben enthalten sie vorgenannte, zwingenden Bestandteile dieser Erfindung.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat-oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 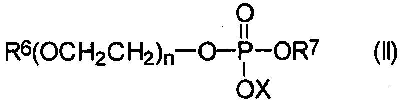 in der R⁶ bevorzugt für einen aliphatischen Kohlenwasserstoffrest mit 8 bis 30 Kohlenstoffatomen, R⁷ für Wasserstoff, einen Rest (CH₂CH₂O)ₙR⁶ oder X, n für Zahlen von 1 bis 10 und X für Wasserstoff, ein Alkali- oder Erdalkalimetall oder NR⁸R⁹R¹⁰R¹¹, mit R⁸ bis R¹¹ unabhängig voneinander stehend für einen C₁ bis C₄ - Kohlenwasserstoffrest, steht,
- sulfatierte Fettsäurealkylenglykolester der Formel (III),

R¹²CO(AlkO)ₙSO₃M (III)

in der R¹²CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Acylglutamate, welche Kondensationsprodukte aus der Glutaminsäure und C8 - C30 Fettsäuren, bevorzugt von Fettsäuren mit 12 bis 18 C-Atomen darstellen und welche in neutralisierter Form als K-, Na- oder Ammoniumsalz vorliegen, und
- Acylaspartate, welche Kondensationsprodukte aus der Asparaginsäure und C8 - C30 Fettsäuren, bevorzugt von Fettsäuren mit 12 bis 18 C-Atomen darstellen und welche in neutralisierter Form als K-, Na- oder Ammoniumsalz vorliegen.
- Acylsarcoside mit 8 bis 24 C-Atomen in der Acylgruppe.

Bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Zitronensäureester mit Fettalkoholethoxylaten, wie beispielsweise das unter dem Handlesnamen "Plantapon LC-7" vetriebene Produkt sowie Natriumtridecethsulfate, beispielsweise das unter den Handelsnamen "Miracare SLB 365", "Genapol XRO" oder "Rhodapex EST-30" vertriebenen Natriumtridecethsulfat-Tensidmischungen.

Besonders bevorzugte anionische Tenside sind die Alkali- oder Ammoniumsalze des Laurylethersulfates mit einem Ethoxylierungsgrad von 2 bis 4 EO.

Das oder die anionische(n) Tensid(e) wird (werden) in den erfindungsgemäßen Mitteln - bezogen auf das gesamte Mittel, bevorzugt in einer Menge von 0,1 bis 35 Gew.-%, besonders bevorzugt in einer Menge von 0,5 bis 25 Gew.% und insbesondere in einer Menge von 1 bis 20 Gew.-% eingesetzt.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylamrnoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Die amphoteren und/oder zwitterionischen Tenside werden in den erfindungsgemäßen Mitteln - bezogen auf das gesamte Mittel, bevorzugt in einer Menge von 0,05 bis 18 Gew.%, besonders bevorzugt in einer Menge von 0,1 bis 15 Gew.% und insbesondere in einer Menge von 0,5 bis 10 Gew.-% eingesetzt

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆- Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl, beispielsweise Rizinusöl-hydriert+40 EO, wie es beispielsweise unter dem Handelsnamen Cremophor CO 455 von der Firma SHC im Handel erhältlich ist,
- Aminoxide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Al-kylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen Tenside werden in den erfindungsgemäßen Mitteln - bezogen auf das gesamte Mittel-, bevorzugt in einer Menge von 0,1 bis 15 Gew.-% und insbesondere in einer Menge von 0,5 bis 10 Gew.-% eingesetzt.

Gemäß einer weiteren Ausführungsform der Erfindung können die tensidhaltigen Hautreinigungs-und/oder -pflegemittel weiterhin kationische Tenside vom Typ der quartären Ammoniumverbindungen, der Esterquats und der Amidoamine enthalten.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammonium-chlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlörid, sowie Dehyquart^{®} F-75, Dehyquart^{®}C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.%, bezogen auf das gesamte Mittel, enthalten. Mengen von 0,1 bis 5 Gew.% sind besonders bevorzugt.

Unter erfindungsgemäß geeigneten kationischen Polymeren sind Polymere zu verstehen, welche in der Haupt- und/oder Seitenkette Gruppen aufweisen, welche "temporär" oder "permanent" kationisch sein können. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (VI), 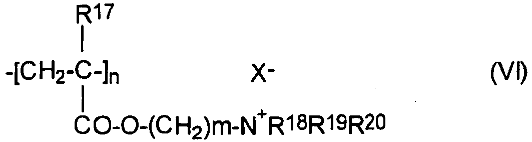 in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 und Salcare^{®} SC 96 im Handel erhältlich.

Copolymere mit Monomereinheiten gemäß Formel (VI) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen erhältlichen Produkte Q2-7224 (Hersteller: Dow Coming; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

In einer besonders bevorzugten Ausführungsform der Erfindung ist als kationisches Polymer mindestens ein Polymer aus der Gruppe Polyquaternium-2, Polyquaternium-7 (Merquat 550), Polyquaternium-6, Polyquaternium-10 und/oder Polyquaternium-67 (SoftCat^{®}-Polymere) in den Mitteln enthalten.

Die kationischen Polymere können in den erfindungsgemäßen Mitteln - bezogen auf das gesamte Mittel - in Mengen von 0,05 bis 5 Gew.-% enthalten sein. Mengen von 0,1 bis 3, insbesondere von 0,15 bis 2 Gew.-%, sind besonders bevorzugt.

Als wasserunlösliche Ölkomponenten eignen sich erfindungsgemäß pflanzliche, mineralische oder synthetische Öle, sowie Gemische dieser Komponenten.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Aprikosenkernöl, Babssuöl, Olivenöl, Weizenkeimöl, Kürbiskernöl, Malvenöl, Haselnussöl, Safloröl, Canolaöl, Sasanquaöl, Jojobaöl und Shea-Butter. Besonders bevorzugt sind Aprikosenkernöl, Pfirsichkernöl, Mandelöl und/oder Olivenöl.

Als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beispielsweise das als Handelsprodukt erhältliche 1,3-Di-(2-ethylhexyl)-cyclohexan (Cetiol^{®} S).

Als synthetische Öle kommen Silikonverbindungen, insbesondere Dialkyl- und Alkylarylsilikone, wie beispielsweise Dimethylpolysiloxan und Methylphenylpolysiloxan, sowie deren hydroxy-terminierte, alkoxylierte und quaternierte Analoga in Betracht. Beispiele für solche Silikone sind die von Dow Corning unter den Bezeichnungen DC 190, DC 200, DC 245, DC 246, DC 344 und DC 345 (Cyclomethicone) vertriebenen Produkte.

Als Ölkomponente kann weiterhin ein Dialkylether dienen.

Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Erfindungsgemäß besonders bevorzugt ist der Di-n-octylether, der im Handel unter der Bezeichnung Cetiol^{®} OE erhältlich ist.

Die erfindungsgemäßen Reinigungsmittel enthalten die wasserunlösliche Ölkomponente bevorzugt in einem Mengenbereich von 0,1 bis 5 Gew.%, insbesondere von 0,5 bis 2 Gew.%, bezogen auf das Gesamtgewicht des Mittels.

In einer weiteren bevorzugten Ausführungsform der Erfindung kann die Wirkung des erfindungsgemäßen Wirkstoffs durch weitere Fettstoffe noch weiter optimiert werden. Unter weiteren Fettstoffen sind zu verstehen Fettsäuren, Fettalkohole sowie natürliche und synthetische Wachse, welche sowohl in fester Form als auch flüssig in wäßriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole werden in Mengen von 0,1 - 20 Gew.%, bezogen auf die gesamte Zubereitung, bevorzugt in Mengen von 0,1 - 10 Gew.% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}). Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-O18, Monomuls^{®} 90-L12, Cetiol^{®} HE oder Cutina^{®} MD.

Die Gesamtmenge an ÖI- und Fettkomponenten in den erfindungsgemäßen Mitteln beträgt üblicherweise 0,01 - 15 Gew.-%, bezogen auf das gesamte Mittel. Mengen von 0,05- 5 Gew.% sind erfindungsgemäß bevorzugt.

Unter erfindungsgemäß bevorzugten Vitaminen, Provitaminen und Vitaminvorstufen sowie deren Derivaten sind solche Vertreter zu verstehen, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,01-1 Gew.-%, bezogen auf die gesamte Zubereitung.

Zur Vitamin B-Gruppe oder zu dem Vitamin B-Komplex gehören u. a.
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate.
Vitamin B₆ (Pyridoxin sowie Pyridoxamin und Pyridoxal).

Die genannten Verbindungen der Vitamin B-Gruppe sind in den erfindungsgemäßen Mitteln bevorzugt in Mengen von 0,01 - 2 Gew.%, bezogen auf das gesamte Mittel, enthalten. Mengen von 0,03 - 1 Gew.-% sind besonders bevorzugt.

Vitamin C (Ascorbinsäure). Vitamin C wird in den erfindungsgemäßen Mitteln bevorzugt in Mengen von 0,01 bis 3 Gew.%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,01-1 Gew.%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.%, insbesondere in Mengen von 0,001 bis 0,01 Gew.% enthalten.

Bevorzugt enthalten die erfindungsgemäßen Zubereitungen Vitamine, Provitamine und Vitaminvorstufen aus den Gruppen A, E, F und H. Selbstverständlich können auch mehrere Vitamine und Vitaminvorstufen gleichzeitig enthalten sein.

Die Gesamt-Einsatzmenge der Vitamine, Provitamine, Vitaminvorstufen sowie deren Derivaten in den erfindungsgemäßen Mitteln beträgt - bezogen auf das Gesamtgewicht des Mittels - 0,01 bis 5 Gew.-%, vorzugsweise 0,02 bis 4 Gew.% und insbesondere 0,05 bis 3 Gew.-%.

Als Proteinhydrolysate im Sinne der Erfindung werden Proteinhydrolysate und/oder Aminosäuren und deren Derivate (H) verstanden. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®}

(Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Die Proteinhydrolysate oder deren Derivate sind in den erfindungsgemäß verwendeten Zubereitungen bevorzugt in Mengen von 0,1 bis 10 Gew.%, bezogen auf das gesamte Mittel, enthalten. Mengen von 0,1 bis 5 Gew.% sind besonders bevorzugt.

Erfindungsgemäß bevorzugt sind Mittel, die Pflanzenextrakte aus grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Vanille, Eibisch, Meristem, Ginseng und Ingwerwurzel enthalten.

Besonders bevorzugt sind erfindungsgemäß die Extrakte aus Grünem Tee, Mandel, Aloe Vera, Kokosnuß, Mango, Aprikose, Limone, Weizen, Vanille, Kiwi und Melone und insbesondere bevorzugt die Extrakte aus Aloe Vera, Vanille und Melone.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders-geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.% Aktivsubstanz und als Lösungsmittel das bei ihrer Gewinnung eingesetzte Extraktionsmittel oder Extraktionsmittelgemisch.

Die Pflanzenextrakte werden in den erfindungsgemäßen Mitteln - bezogen auf ihr Gewicht - in einer Menge von 0,01 bis 5 Gew.-%, vorzugsweise 0,02 bis 4 Gew.% und insbesondere 0,05 bis 3 Gew.-% eingesetzt.

Weiterhin enthalten die erfindungsgemäßen Zusammensetzungen in einer bevorzugten Form, Perlglanzwachse. Als Perlglanzwachse kommen vorzugsweise Acylierungsprodukte von Glycolen und deren Eigenkondensationsprodukten in Frage, die der folgenden Formel Perl-1 folgen, 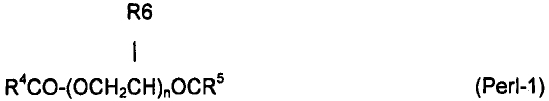 in der R⁴CO und R⁵CO unabhängig von einander für gesättigte oder ungesättigte Acylreste mit 8 bis 22, vorzugsweise 12 bis 18 Kohlenstoffatomen, R⁶ für Wasserstoff oder eine Methylgruppe und n für Zahlen von 1 bis 5 steht. Vorzugsweise kommen für diesen Zweck Acylierungsprodukte des Ethylenglycols mit Kokosfettsäure oder Stearinsäure, wie insbesondere Ethylenglycoldistearat in Betracht.

Eine weitere Gruppe von Perlglanzwachsen stellen Fettsäurepartialester dar, die der Formel (Perl-2) folgen, 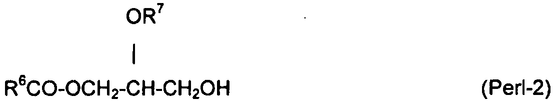 in der R⁶CO für einen gesättigten oder ungesättigten Acylrest mit 6 bis 22, vorzugsweise 12 bis 18 Kohlenstoffatomen und R⁷ für Wasserstoff oder einen Acylrest R⁶CO steht. Typische Beispiele sind Mono- und Diglyceride sowie deren technischen Gemische, die herstellungsbedingt noch geringe Anteile an Trifettsäureestern enthalten können. Gegenüber den acylierten Glycolen weisen sie den Vorteil auf, daß sie keine Ethylenoxideinheiten enthalten, was von verschiedenen Herstellern kosmetischer Zubereitungen bevorzugt wird. In einer bevorzugten Ausführungsform der Erfindung werden daher beispielsweise Mono/Diglyceridgemische eingesetzt, die sich von der Laurinsäure, der Kokosfettsäure, der Palmitinsäure, der Öl-säure und insbesondere der Stearinsäure ableiten.

Anstatt eines relativ teuren Perlglanzwachses zu verwenden, kann erfindungsgemaß auch ein Trübungsmittel verwendet werden. Als Trübungsmittel werden im allgemeinen polymere Dispersionen von Styrol, Styrol-Butadien und ähnlichen Substanzen verwendet. Auch derartige Dispersionen sind dem Fachmann bekannt und im Handel erhältlich.

Die Perlglanz - und Trübungsmittel werden in den erfindungsgemäßen Zusammensetzungen üblicherweise in einer Menge von 0,01 bis 10 Gew.% verwendet. Mengen von 0,05 bis 5 Gew. % sind hierbei bevorzugt.

Des Weiteren können die erfindungsgemäßen Rinse-off-Produkte fakultativ Stoffe aus der Gruppe der Lösungsvermittler, der Parfüme und der Konservierungsmittel enthalten. Diese Stoffgruppen werden im Rahmen der Leave-on-Produkte näher beschrieben.

Selbstverständlich können alle Stoffe, die explizit im Rahmen der Rinse-off-Produkte beschrieben wurden, auch in einem Leave-on-Produkt enthalten sein und alle Stoffe, die explizit im Rahmen der Leave-on-Produkte beschrieben wurden können auch in einem Rinse-off-Produkt enthalten sein.

In einer zweiten bevorzugten Ausführungsform der Erfindung handelt es sich bei dem Hautreinigungs oder -plegemittel um ein Leave-on-Produkt wie eine wässrige, alkoholische oder wässriglalkoholische Dispersion, eine Öldispersion, eine Salbe, eine Creme, eine Emulsion, eine Suspension, eine Milch, eine Paste, ein Gel, ein Schaum oder ein Spray, oder ein Substrat, das mit dem Mittel imprägniert, getränkt oder beschichtet wurde.

In einer bevorzugten der oben genannten Ausführungsformen liegen die erfindungsgemäßen Mittel in flüssiger Form vor. Zum Erreichen einer flüssigen Konsistenz kann unter Umständen der Einsatz sowohl flüssiger organischer Lösungsmittel, wie auch der von Wasser angezeigt sein. Die erfindungsgemäßen Mittel enthalten daher gegebenenfalls Lösungsmittel.

Lösungsmittel, die in den erfindungsgemäßen Mitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethyl-, -ethyl- oder -propyl-ether, Butoxypropoxy-propanol (BPP), Dipropylenglykolmonomethyl-, oder -ethylether, Diisopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether, Diacetin, Triethylcitrat sowie Mischungen dieser Lösungsmittel.

Zu den Alkoholen, die in der vorliegenden Erfindung als Cosolventien eingesetzt werden können, gehören flüssige Polyethylenglykole, mit niederem Molekulargewicht, beispielsweise Polyethylenglykole mit einem Molekulargewicht von 200, 300, 400 oder 600. Weitere geeignete Cosolventien sind andere Alkohole, zum Beispiel (a) niedere Alkohole wie Ethanol, Propanol, Isopropanol und n-Butanol, (b) Ketone wie Aceton und Methylethylketon, (c) C₂-C₄-Polyole wie ein Diol oder ein Triol, beispielsweise Ethylenglykol, Propylenglykol, Glycerin oder Gemische davon.

Die Zusammensetzungen dieser zweiten bevorzugten Ausführungsform enthalten weiterhin ein Öl, das vorzugsweise ebenfalls antiseptisch wirksam ist, vorzugsweise ein etherisches Öl.

Bei diesem antiseptisch wirksamen Öl handelt es sich vorzugsweise um etherisches Öl, das insbesondere ausgewählt ist aus der Gruppe der Angelica fine - Angelica archangelica, Anis - Pimpinella Anisum, Benzoe siam - Styrax tokinensis, Cabreuva - Myrocarpus fastigiatus, Cajeput - Melaleuca leucadendron, Cistrose - Cistrus ladaniferus, Copaiba-Balsam - Copaifera reticulata, Costuswurzel - Saussurea discolor, Edeltannennadel - Abies alba, Elemi - Canarium luzonicum, Fenchel - Foeniculum dulce Fichtennadel - Picea abies, Geranium - Pelargonium graveolens, Ho-Blätter - Cinnamonum camphora, Immortelle (Strohblume) Helichrysum ang., Ingwer extra - Zingiber off., Johanniskraut - Hypericum perforatum, Jojoba, Kamille deutsch - Matricaria recutita, Kamille blau fine - Matricaria chamomilla, Kamille röm. - Anthemis nobilis, Kamille wild - Ormensis multicaulis, Karotte - Daucus carota, Latschenkiefer - Pinus mugho, Lavandin - Lavendula hybrida, Litsea Cubeba - (May Chang), Manuka - Leptospermum scoparium, Melisse - Melissa officinalis, Meerkiefer - Pinus pinaster" Myrrhe - Commiphora molmol, Myrthe - Myrtus communis, Neem - Azadirachta, Niaouli - (MQV) Melaleuca quin. viridiflora, Palmarosa - Cymbopogom martini, Patchouli - Pogostemon patschuli, Perubalsam - Myroxylon balsamum var. pereirae, Raventsara aromatica, Rosmarin Rosmarinus officinalis, Rosenholz - Aniba rosae odora, Salbei - Salvia officinalis Schachtelhalm - Equisetaceae, Schafgarbe extra - Achillea millefolia, Spitzwegerich - Plantago lanceolata, Styrax - Liquidambar orientalis, Tagetes (Ringelblume) Tagetes patula, Teebaum - Melaleuca alternifolia, Tolubalsam - Myroxylon Balsamum L., Virginia-Zeder - Juniperus virginiana, Weihrauch (Olibanum) - Boswellia carteri, Weißtanne - Abies alba.

Darüber hinaus können die Öle dieser zweiten, bevorzugten Ausführungsform selbstverständlich auch aus synthetischen Ölen, wie sie bereits im Rahmen der Rinse-off-Produkte beschrieben wurden, ausgewählt sein.

Weiterhin zeichnet sich das erfindungsgemäße Mittel dieser zweiten Ausführungsform dadurch aus, daß es terpenhaltige Pflanzen extrakte enthält, bevorzugt Extrakte aus Pflanzen der Gattungen Leptospermum und Melaleuca aus der Familie der Myrtaceae und besonders bevorzugt Teebaumöl (Extrakt von Melaleuca alternifolia) oder Öl des Kajeputbaums (Melaleuca leucadendra). Die dabei vorzugsweise eingesetzten Extrakte aus den Blättern bzw. Pflanzenteilen der Pflanzengattungen Leptospermum und Melaleuca, deren natürliches Verbreitungsgebiet sich auf die subtropischen Küstenregionen von New South Wales beschränken, werden durch Wasserdampfdestillation bzw. Extraktion gewonnen. Dabei dienen besonders bevorzugt Blätter des australischen Teebaums (Melaleuca alternifolia) als Ausgangsprodukte. In einer bevorzugten Ausführungsform zeichnet sich das erfindungsgemäße Mittel dadurch aus, daß der terpenhaltige Pflanzenextrakt Teebaumöl ist und in einer Menge von 0,005-2 Gew.% in dem Mittel enthalten ist.

Die genannten Öle, Hanföl, Borretschöl, Echiumöl, Trichodesmaöl, Nachtkerzenöl, das Kernöl der schwarzen Johannisbeere und/oder Schwarzkümmelöl, sind hautschützende und/oder hautheilende Aktivstoffe im Sinne der Erfindung.

In einer weiteren besonderen Ausführungsform gelangt neben einem oder mehreren dieser Öle (Hanföl, Borretschöl, Echiumöl, Trichodesmaöl, Nachtkerzenöl, Kernöl der schwarzen Johannisbeere und/oder Schwarzkümmelöl) ein zusätzlicher hautschützender Stoff zur Anwendung. Bei diesem hautschützenden Stoff handelt es sich vorteilhafterweise um ein hautschützendes Öl, z. B. auch um ein Trägeröl, insbesondere ausgewählt aus der Gruppe Algenöl Oleum Phaeophyceae, Aloe-Vera Öl Aloe vera brasiliana, Aprikosenkernöl Prunus armeniaca, Arnikaöl Arnica montana, Avocadoöl Persea americana, Calendulaöl Calendula officinalis, Camelliaöl Camellia oleifera, Distelöl Carthamus tinctorius, Erdnuß-öl Arachis hypogaea, Haselnußöl Corylus avellana/, Johanniskrautöl Hypericum perforatum, Jojobaöl Simondsia chinensis, Karottenöl Daucus carota, Kokosöl Cocos nucifera, Kürbiskernöl Curcubita pepo, Kukuinußöl Aleurites moluccana, Macadamianußöl Macadamia ternifolia , Mandelöl Prunus dulcis, Meadowfoamöl Limnanthes alba, Olivenöl Olea europaea, Pfirsichkernöl Prunus persica, Rapsöl Brassica oleifera, Rizinusöl Ricinus communis, Sesamöl Sesamium indicum, Sonnenblumenöl Helianthus annus, Traubenkernöl Vitis vinifera, Walnußöl Juglans regia, Weizenkeimöl Triticum sativum, Gold Of Pleasure Camelina Sativa, Kemiriöl Aleurites moluccana, Passionsblumenöl Passiflora Incarnata, Reiskeimöl Oryza Sativa.

Alle die gerade aufgeführten Öle sind natürliche Emollientien, d. h. Mittel, die Körpergewebe weicher und geschmeidiger machen und die Rauhigkeit der Haut vermindern. Diese Öle wirken also zum einen hautpflegend. Zum anderen weisen gerade diese Öle weitere spezifische Wirkungen auf, die ein synergistisches Zusammenwirken mit der Haut und deren Selbstregulierungskräften nach sich ziehen und einen Schutz auch unter widrigen Bedingungen ermöglichen.

In einer bevorzugten Ausführungsform enthalten die erfindungsgemäßen Mittel dieser zweiten bevorzugten Ausführungsform einen Mindestgehalt aller oben genannter Ölkomponente(n) von 1 Gew.-% vorzugsweise 2 Gew.-%, insbesondere 2,5 Gew.-%, bevorzugt 3 Gew.-%, besonders bevorzugt 3,5 Gew.-%, jeweils bezogen auf das gesamte Mittel.

In einer bevorzugten Ausführungsform liegt das erfindungsgemäße Mittel dieser zweiten bevorzugten Ausführungsfrom in flüssiger Form, insbesondere emulgiert vor. Dabei beträgt der Wassergehalt vorzugsweise zwischen 5 und 95 Gew.%, bevorzugt 15 bis 75 Gew.% und insbesondere 40 bis 65 Gew.% jeweils bezogen auf das Mittel.

In einer weiteren bevorzugten Ausführungsform enthalten die erfindungsgemäßen Mittel dieser zweiten Ausführungsform zusätzlich Milchsäure und/oder deren Salze und/oder Citronensäure und/oder deren Salze, insbesondere deren Natrium- und/oder Kaliumsalze. Diese beiden hautfreundlichen Säuren und/oder deren Salze, besonders die Milchsäure und ihre Salze, dienen u. a. dazu, den natürlichen Säureschutzmantel bzw. Hydrolipidfilm der Haut zu unterstützen bzw. zu erneuern.

Bevorzugt liegt der pH-Wert des erfindungsgemäßen Mittels dieser zweiten Ausführungsform im Bereich zwischen 2,5 und 11 und insbesondere zwischen 4 und 9, gemessen bei einer Temperatur von 20°C insbesondere an einer 1 %-igen wässrigen Lösung bzw. Dispersion des Mittels. Dies entspricht dem pH-Wert der Haut eines gesunden Menschen.

Neben den genannten besonderen Feuchthaltefaktoren wie beispielsweise Harnstoff und Milchsäure und deren Salzen können die erfindungsgemäßen Mittel dieser zweiten bevorzugten Ausführungsform weitere Feuchthaltefaktoren beinhalten beispielsweise solche, die ausgewählt sind aus folgender Gruppe: Aminosäuren, Chitosan oder Chitosansalze/- derivate, Ethylenglykol, Glucosamin, Glycerin, Diglycerin, Triglycerin, Harnsäure, Honig und gehärteter Honig, Kreatinin, Hydrolyseprodukte des Kollagens, Lactitol, Polyole und Polyolderivate (beispielsweise Butylenglycol, Erythrit, Propylenglycol, 1,2,6-Hexantrlol, Polyethylenglycole wie PEG-4, PEG-6, PEG-7, PEG-8, PEG-9, PEG-10, PEG-12, PEG-14, PEG-16, PEG-18, PEG-20), Pyrrolidoncarbonsäure Zucker und Zuckerderivate (beispielsweise Fructose, Glucose, Maltose, Maltitol, Mannit, Inosit, Sorbit, Sorbitylsilandiol,-Suerose, Trehalose, Xylose, Xylit, Glucuronsäure und deren Salze), ethoxyliertes Sorbit (Sorbeth-6, Sorbeth-20, Sorbeth-30, Sorbeth-40), gehärtete Stärkehydrolysate sowie Mischungen aus gehärtetem Weizenprotein und PEG-20-Acetatcopolymer, insbesondere aber Panthenol.

Das erfindungsgemäße Mittel dieser zweiten bevorzugten Ausführungsform kann zusätzlich zu den heilenden Aktivstoffen einen oder mehrere desodorierende Wirkstoffe enthalten.

Gemäß einer weiteren bevorzugten Ausführungsform der Erfindung enthalten die Zusammensetzungen ferner einen Lösungsvermittler. Dieser ist bevorzugt nichtionisch und hydrophil. Erfindungsgemäß bevorzugte nichtionische hydrophile Lösungsvermittler sind ausgewählt aus den Anlagerungsprodukten von 4 bis 100 Ethylenoxid-Einheiten an gegebenenfalls gehärtete Mono-, Di- und Triglyceride von C₈₋₂₂-Fettsäuren, den Anlagerungsprodukten von 5 bis 40 Ethylenoxid-Einheiten an C₈₋₂₂-Fettalkohole sowie den Anlagerungsprodukten von 2 bis 50 Ethylenoxid-Einheiten und 2 bis 35 Propylenoxid-Einheiten an C₃-C₅-Alkanole. Beispiele für ethoxylierte Mono-, Di- und Triglyceride von C₈₋₂₂-Fettsäuren mit 4 bis 60 Ethylenoxid-Einheiten sind hydriertes ethoxyliertes Castoröl (INCI-Bezeichnung z.B. PEG-40 Hydrogenated Cator Oil), Olivenölethoxylat (INCI-Bezeichnung: PEG-10 Olive Glycerides); Mandelölethoxylat, Nerzölethoxylat, Polyoxyethylenglycolcapryl-/caprinsäure-glyceride, Polyoxyethylenglycerinmonolaurat und Polyoxyethylenglycolkokosfettsäureglyceride. Beispiele für geeignete ethoxylierte C₈₋₂₂-Fettalkohole sind Laureth-12, Laureth-23, Trideceth-8, Ceteareth-12, Ceteareth-15, Ceteareth-20, Ceteareth-30, Steareth-10, Steareth-15, Steareth-20, Steareth-30, Steareth-40, Oleth-10 oder Oleth-20. Beispiele für bevorzugte Polyethylenglycol-Polypropylenglycol-Mischether von C₃-C₅-Alkanolen sind die PEG-PPG-Addukte von 1-Propanol, 2-Propanol und iso-Propanol, 1-Butanol, 2-Butanol, iso-Butanol und 1-Pentanol, 2-Pentanol und Amylalkohol mit 2 - 50, bevorzugt 4 - 40 Ethylenoxid-Einheiten und 2 - 35, bevorzugt 4 - 30 Propylenoxid-Einheiten, insbesondere PPG-28-Buteth-35, PPG-26-Buteth-26, PPG-5-Buteth-5, PPG-25-Buteth-25, PPG-5-Buteth-20, PPG-33-Buteth-45, PPG-20-Buteth-30 oder PPG-12-Buteth-16. Bevorzugte erfindungsgemäße Zusammensetzungen enthalten den nichtionischen hydrophilen Lösungsvermittler in Mengen von 0,1 - 7 Gew.%, besonders bevorzugt 0,5 - 6 Gew.-% und außerordentlich bevorzugt 1 - 4 Gew.%, jeweils bezogen auf die gesamte Zusammensetzung.

In einer bevorzugten Ausführungsform liegt das erfindungsgemäße Mittel in Form einer Emulsion vor. Bei Emulsionen handelt es sich um disperse Systeme von mindestens zwei nicht mischbaren Flüssigkeiten, wobei die eine Phase in Form feiner Tröpfchen in der anderen, zusammenhängenden Phase verteilt ist. Man unterscheidet hierbei zwischen Makro- und Mikroemulsionen, wobei diese Erfindung alle Emulsionsgattungen umfaßt.

Die Herstellung der erfindungsgemäßen Emulsionen erfolgt nach den klassischen Vorgehensweisen, z. B. durch Schütteln, Schlagen, Rühren, turbulentes Mischen, Einspritzen einer Flüssigkeit in eine andere, durch Emulgierzentrifugen, Kolloidmühlen, Homogenisatoren, beispielsweise Inline-Maschinen, die nach dem Rotor/ Stator-Prinzip arbeiten oder Hochdruck-Homogensiatoren, die durch plötzliche Druckentspannung emulgieren, durch Schwingungen und Kavitation in der Mischung und vielen andere mehr. Nach neusten Untersuchungen lassen sich auch statische Mischer einsetzen, vorzugsweise moderne Mikromischer.

Die Emulsionen können dabei mit sogenannten Emulgatoren oder Stabilisatoren stabilisiert werden, so daß ein mögliches Aufrahmen oder Sedimentieren oder die Neigung der dispergierten Teilchen zur Zusammenballung erschwert ist. Solche Verbindungen haben für gewöhnlich einen amphiphilen Charakter, weisen also mindestens eine polare sowie eine unpolare Gruppe auf, wobei es auch ein Übergewicht der apolaren Gruppe geben kann, man spricht dann von Co-Emulgatoren. Als unpolare Gruppen gelangen in der Regel gesättigte oder ungesättigte, verzweigte oder unverzweigte Alkylreste sowie Aryl- bzw. Alkylaryl-Reste zur Anwendung. Als polare Endgruppen treten Carboxylat, Sulfonat, Sulfat, Phosphat, Polyphosphat, Lactat, Citrat , Tartrat, Amin-Salze, quartäre Ammoniumverbindungen, Betaine, Alkohol-, Polyether,- Glycerin-, Sorbit-, Pentaerythrit-, Saccharose-, Essigsäure-, Milchsäure-Reste auf, als polare Zwischengruppen fungieren Hydroxy-, Ester-, Sulfamid-, Amid-, Polyamid-, Polyamin-, Amin-, Ether-, Polyether-, Glycerin-, Sorbit-, Pentaerythrit und Saccharose-Gruppen, um nur wenige zu nennen. Man unterscheidet ganz allgemein zwischen anionischen, kationischen, amphoteren und zwitterionischen sowie nichtionischen Emulgatoren, wobei im Rahmen dieser Erfindung prinzipiell alle dieser Emulgatoren zur Anwendung gelangen können und auch solche, die nicht in die vorgenannten Kategorien passen. Zu beachten ist im Rahmen dieser Erfindung jedoch eine physiologische und toxikologische Unbedenklichkeit des Emulgators bei erfindungsgemäßer Verwendung, die im Einklang mit dem Sinn der Erfindung steht, nämlich der Haut einen Vorteil zu verschaffen. Bevorzugt werden nichtionische Emulgatoren verwendet. Auch alle erdenklichen und üblichen Emulgierhilfsstoffe können erfindungsgemäß vorteilhafterweise zur Anwendung gelangen.

Liegt die Ölphase fein verteilt in der Wasserphase vor, so handelt es sich um eine Öl-in-Wasser-Emulsion (O/W-Emulsion, z. B. Milch), was erfindungsgemäß bevorzugt ist. Der Grundcharakter einer O/W-Emulsion ist durch das Wasser geprägt, d. h. sie wirkt im allgemeinen weniger fettend auf der Haut, ist eher mattierend und zieht schneller in die Haut ein als eine W/O-Emulsion, was vorteilhaft sein kann.

Erfindungsgemäße Mittel lassen sich vorteilhafterweise als stabile O/W-Zubereitungen zur erfindungsgemäßen dermatologischen Anwendung formulieren, beispielsweise in Form von Cremes und Salben, die im Bereich von Raum- bis Hauttemperatur streichfähig sind, oder , was bevorzugt ist, als Lotionen oder als Milch, die in diesem Temperaturbereich eher fließfähig sind.

O/W-Emulsionen gemäß der vorliegenden Erfindung enthalten, wenn es aus Stabilitätsgründen erforderlich ist, einen oder mehrere Emulgatoren, vorzugsweise ausgewählt aus der Gruppe der folgenden Substanzen, die für gewöhnlich als W/O-Emulgatoren wirken:

Lanolin, mikrokristallines Wachs (Cera microcristallina) im Gemisch mit Paraffinöl (Paraffinum liquidum), Ozokerit, hydriertes Ricinusöl, Polyglyceryl-3-Oleat, Wollwachssäuregemische, Wollwachsalkoholgemische, Pentaerythrithylisostearat, Polyglyceryl-3-Diisostearat, Bienenwachs (Cera alba) und Stearinsäure, Natriumdihydroxycetylphosphat im Gemisch mit Isopropylhydroxycetylether, Methylglucosedioleat, Methylglucosedioleat im Gemisch mit Hydroxystearat und Bienenwachs, Mineralöl im Gemisch mit Petrolatum und Ozokerit und Glyceryloleat und Lanolinalkohol, Petrolatum im Gemisch mit Ozokerit und hydriertem Ricinusöl und Glycerylisostearat und Polyglyceryl-3-oleat, PEG-7- hydriertes Ricinusöl, Ozokerit und hydriertes Ricinusöl, Polyglyceryl-4- isostearat, Polyglyceryl-4-isostearat im Gemisch mit Cetyldimethiconcopolyol und Hexyllaurat, Laurylmethiconcopolyol, Cetyldimethiconcopolyol, Acrylat/C10-C30-Alkylacrylat- Crosspolymer, Poloxamer 101, Polyglyceryl-2-dipolyhydroxystearat, Polyglyceryl-3-Diisostearat, Polyglyceryl-4-dipolyhydroxystearat, PEG-30- dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

In einer bevorzugten Ausführungsform enthalten die erfindungsgemäßen Mittel als Emulgator Cetylstearylalkohol mit ca. 30 Ethoxy-Einheiten, vorzugsweise Eumulgin B3 (Cetylstearylalkohol + 30 Ethoxy-Einheiten), oder ethoxylierte Fettsäure-alkanolamide, wie Kokosfettsäuremonoethanolamid + 4 Ethoxy-Einheiten, was Eumulgin C4 entspricht (beide : Cognis Deutschland GmbH).

Die Gesamtmenge an erfindungsgemäß verwendeten Emulgatoren in den erfindungsgemäßen Zubereitungen wird vorteilhaft aus dem Bereich von 0,05 bis 10,0 Gew.-%, bevorzugt 0,1 bis 5,0 Gew.-% gewählt, bezogen auf das Gesamtgewicht der Zubereitungen.

Besonders vorteilhaft im Sinne der vorliegenden Erfindung sind sprühbare O/W-Emulsionen, insbesondere O/W-Mikroemulsionen.

Im Sinne der Erfindung sind sowohl Makro- als auch Mikroemulsionen in vorteilhafterweise einsetz- und herstellbar.

Die erfindungsgemäßen kosmetischen und dermatologischen Zubereitungen können gegebenfalls weitere kosmetische Wirk-, Hilfs- und/oder Zusatzstoffe enthalten, wie sie üblicherweise in solchen Zubereitungen verwendet werden und dem Fachmann aus dem Stand der Technik gut bekannt sind, beispielsweise Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch hierbei ist es selbstverständlich jeweils zu gewährleisten daß die zusätzlichen Bestandteile dem Anliegen der Erfindung, das Wohlergehen der Haut zu fördern, nicht entgegenwirken.

Die erfindungsgemäßen Metalloxidpartikel werden durch Licht aktiviert. Es ist daher bevorzugt, dass die erfindungsgemäßen Zubereitungen keine Antioxidantien enthalten. Für einige Ausführungsformen kann es dennoch von Vorteil sein, dass ihnen ein geringer Gehalt an Antioxidantien zugegeben wird. Ein dritter Gegenstand der Erfindung ist die kosmetische Verwendung des/der erfindungsgemäßen Mittel(s) zur Prophylaxe, Verminderung, Beseitigung und/oder Linderung von bakteriellen, fungiziden und/oder viralen Infektionen.

Ein weiterer Gegenstand der Erfindung ist ein Erzeugnis, enthaltend ein erfindungsgemäßes Mittel in flüssiger Form, insbesondere dispergiert, und eine Applikationsvorrichtung, vorzugsweise Sprühapplikator, Airfoamer, Roller und/oder Dosierspender, mit welcher das Mittel auf die Haut aufgebracht werden kann. Bevorzugt ist der Sprühspender ein manuell aktivierbarer Sprühspender, insbesondere ausgewählt aus der Gruppe, umfassend Aerosolsprühspender, selbst Druck aufbauende Sprühspender, Pumpsprühspender und Triggersprühspender, insbesondere Pumpsprühspender und Triggersprühspender mit einem Behälter aus Polyethylen oder Polyethylenterephthalat. Solche und ähnliche Sprühspender oder damit verwandte Applikationsvorrichtungen sind handelsüblich und sämtliche handelsüblichen Sprühspender oder verwandte Applikationsvorrichtungen kommen zur erfindungsgemäßen Applikation in Betracht.

Die folgenden Beispiele sollen die Erfindung näher erläutern, ohne sie darauf zu beschränken.

Die aufgeführten Mengenangaben in den Beispielen beziehen sich, sofern nicht anders angegeben, auf Gew.-%.

### Beispiele:

### 1) Rinse-off-Formulierungen

### a) Bodywash

| **Handelsprodukt** | **Einsatzmenge in Gew.-%** |
|---|---|
| Texapon^{®1} N 70 | 12,00 |
| Dehyton^{®2} PK 45 | 6,00 |
| Polymer^{®3} JR 400 | 0,25 |
| Plantacare^{®4} 818 | 2,00 |
| Sodium Salicylate | 0,20 |
| Sodium Benzoate | 0,20 |
| Eumulgin^{®5} HRE 40 | 0,50 |
| Parfum | 0,50 |
| Sodium Chloride | 0,30 |
| Keltrol^{®6} SF | 0,30 |
| Titandioxid^{®7} VLP 7000 | 5,00 |
| pH adjustment, Emollient, Care additives Chelating agent | nach Bedarf |
| Water | ad 100 |

### b) Bar Soaps

| **Handelsprodukt** | **Einsatzmenge in Gew.-%** | | | |
|---|---|---|---|---|
| | **A** | **B** | **C** | **D** |
| Sodium Tallowate | 55,00 | | 58,00 | |
| Sodium Palmitate | | 55,00 | | 50,00 |
| Sodium Cocoate | 22,00 | | 27,00 | |
| Sodium Palm Oleate | | 22,00 | | 27,00 |
| Talc | 5,00 | 2,00 | | |
| Lauryl Glucoside | | 2,00 | | 2,00 |
| Parfum | 1,00 | 1,00 | 1,50 | 0,50 |
| Sodium Chloride | 0,50 | 0,50 | 0,50 | 0,50 |
| Tetrasodium EDTA | 0,30 | 0,20 | 0,30 | 0,10 |
| Tocopherol | 0,10 | 0,30 | | |
| Antibacterial Active | 0,30 | | 0,50 | |
| Farbstoff | 0,01 | 0,05 | 0,03 | |
| Ethylhexylglycerin | | 0,30 | | 0,30 |
| Titandioxid^{®7} VLP 7000 | 5,00 | 8,00 | 2,00 | 10,00 |
| Phenoxyethanol | | | 1,00 | 1,00 |
| Water | ad 100 | ad 100 | ad 100 | ad 100 |

### c) Liquid Handsoap

| **Handelsprodukt** | **Einsatzmenge in Gew.-%** |
|---|---|
| Texapon^{®1} N 70 | 15,00 |
| Dehyton^{®2} PK 45 | 2,00 |
| Polyquart^{®16} 701/NA | 0,80 |
| Plantacare^{®17} 1200 | 1,00 |
| Cetiol^{®18} HE | 0,80 |
| Arlypon^{®19} F-T90 | 1,50 |
| Salicylic Acid | 0,50 |
| Sodium Benzoate | 0,40 |
| Eumulgin^{®5} HRE 40 | 0,50 |
| Parfum | 0,50 |
| Sodium Chloride | 0,30 |
| Keltrol^{®6} SF | 0,30 |
| Titandioxid^{®7} VLP 7000 | 5,00 |
| pH adjustment, Emollient, Care additives Chelating agent | nach Bedarf |
| Water | ad 100 |

### 2) Leave-on-Formulierungen

### a) Hydroalkoholische Dispersion

| **Handelsprodukt** | **Einsatzmenge in Gew.-%** |
|---|---|
| Ethanol | 30,00 |
| Keltrol^{®6} SF | 0,30 |
| Titandioxid^{®7} VLP 7000 | 5,00 |
| Irgasan^{®8} DP 300 | 0,20 |
| Eumulgin^{®9} B3 | 2,00 |
| Parfum | 0,10 |
| Sodium Chloride | 0,30 |
| pH adjustment, Emollient, Care additives Chelating agent | nach Bedarf |
| Water | ad 100 |

### b) Hydroalkoholische Dispersion

| **Handelsprodukt** | **Einsatzmenge in Gew.-%** |
|---|---|
| Ethanol | 30,00 |
| Carbopol^{®10} ETD 2020 | 1,00 |
| Titandioxid^{®7} VLP 7000 | 5,00 |
| Irgasan^{®8} DP 300 | 0,20 |
| Eumulgin^{®9} B3 | 2,00 |
| Parfum | 0,10 |
| Chelating agent | 0,20 |
| pH adjustment, Emollient, Care additives | nach Bedarf |
| Water | ad 100 |

### c) Emulsion

| **Handelsprodukt** | **Einsatzmenge in Gew.-%** |
|---|---|
| Cetiol^{®11} SN | 4,50 |
| Paraffinum Liquidum | 5,00 |
| Cutina^{®12} CBS | 2,50 |
| Edenor^{®13} L2SM | 1,50 |
| Eumulgin^{®9} B3 | 1,00 |
| Mandelöl, süß | 3,00 |
| Propylparaben | 0,30 |
| Pemulen^{®14} TR1 | 0,30 |
| Glycerin 86% pflanzlich | 5,00 |
| Kaliumhydroxid 85% | 0,26 |
| Milchsäure 80% DAB | 0,23 |
| Propandiol-1,2 | 3,00 |
| Methylparaben | 0,30 |
| Parfum | 0,20 |
| Benecell^{®15} MP 333C | 0,30 |
| Phenoxyethanol | 0,80 |
| Titandioxid^{®7} VLP 7000 | 5,00 |
| Water | ad 100 |

### 3) Rinse-off Cleansing towel

Papier, Cellulose oder Textil auf der Basis natürlicher und/oder synthetischer Fasern getränkt mit einer der Formulierungen unter Punkt 2).

### Verwendete Handelsprodukte:

- 1: Sodium Lauryl ether (2EO)sulfate (INCI-Bezeichnung: Sodium Laureth Sulfate); AS: 68-73%; Cognis
- 2: INCI-Bezeichnung: Cocamidopropylbetaine; AS: ca. 40%; Cognis
- 3: INCI-Bezeichnung: Polyquatzernium-10; Amerchol
- 4: C₈-C₁₆ Alkylpolyglucoside; INCI-Bezeichnung: Coco Glucoside; AS: ca. 50%; Cognis
- 5: INCI-Bezeichnung: PEG-40 Hydrogenated Castor Oil; Cognis
- 6: INCI-Bezeichnung: Xanthan Gum; CP Kelco
- 7: Activated Titandioxide; Kronos
- 8: INCI-Bezeichnung: Triclosan; Ciba
- 9: C₁₆-C₁₈-Fettalkohole, ethoxyliert (30 EO); INCI-Bezeichnung: Ceteareth-30; Cognis
- 10: Acrylic Acid Copolymer; INCI-Bezeichnung: Acrylates/C₁₀-C₃₀ Alkyl Acrylate Crosspolymer; Noveon
- 11: INCI-Bezeichnung: Cetearyl Isononanoate; Cognis
- 12: INCI-Bezeichnung: Cetearyl Alcohol, Cetyl Palmitate, Glyceryl Stearate, Coco-Glycerides; Cognis
- 13: INCI-Bezeichnung: Palmitic Acid, Stearic Acid; Cognis
- 14: Crosslinked Acrylic Acid Copolymer; INCI-Bezeichnung: Acrylates/C₁₀-C₃₀ Alkyl Acrylate Crosspolymer; Noveon
- 15: INCI-Bezeichnung: Hydroxypropyl Methylcellulose; Hercules
- 16: Dimethyldiallylammonium Chloride Acrylamide Copolymer; INCI-Bezeichnung: Polyquaternium-7; Cognis
- 17: C₁₂-C₁₆ Fatty alcohol glycoside; INCI-Bezeichnung: Lauryl Glucoside; Cognis
- 18: Glyceryl Monococoate, ethoxylated (7 EO); INCI-Bezeichnung: PEG-7 Glyceryl Cocoate; Cognis
- 19: Fatty alcohols (C₁₂-C₁₄), ethoxylated (2,5 EO); INCI-Bezeichnung: Laureth-2; Cognis

## Patentansprüche

1. Antiseptisches kosmetisches Hautreinigungs- und/oder -pflegemittel, **dadurch gekennzeichnet, dass** es einen lichtaktiven Wirkstoff auf der Basis eines mit Kohlenstoff modifizierten Metalloxids enthält, der eine spezifische Oberfläche nach BET von 200 bis 350 m²/g aufweiset.

2. Mittel nach Anspruch 1, **dadurch gekennzeichnet, dass** der Kohlenstoffgehalt des mit Kohlenstoff modifizierten Metalloxids im Bereich von 0,01 bis 10 Gew.-%, bevorzugt im Bereich von 0,05 bis 5 Gew.-%, besonders bevorzugt im Bereich von 0,3 bis 1,5 Gew.-% und insbesondere im Bereich von 0,4 bis 0,8 Gew.-% liegt.

3. Mittel nach einem der Ansprüche 1 oder 2, **dadurch gekennzeichnet, dass** die Teilchengröße des modifizierten Metalloxids in Bereich von 2 bis 600 nm und bevorzugt im Bereich von 200 bis 400 nm liegt.

4. Mittel nach einem der Ansprüche 1 bis 3, **dadurch gekennzeichnet, dass** es - bezogen auf das gesamte Mittel - 0,01 bis 20 Gew.-%, bevorzugt 0,05 bis 10 Gew.-% und insbesondere 0,1 bis 5 Gew.-% des modifizierten Metalloxids enthalt.

5. Mittel nach einem der Ansprüche 1 bis 4, **dadurch gekennzeichnet, dass** das Metalloxid ausgewählt ist aus Zinkoxid und/oder Titanoxid, insbesondere aus Titanoxid.

6. Mittel nach einem der Ansprüche 1 bis 5, **dadurch gekennzeichnet, dass** es als Rinse-off-Produkt wie eine Handwaschseife, ein Flüssighandwaschmittel, eine Seife, ein Syndet, ein Duschgel oder ein Duschbad, oder als Leave-on-Produkt wie eine wässrige, alkoholische oder wässrig/alkoholische Dispersion, eine Oldispersion, eine Salbe, eine Creme, eine Emulsion, eine Suspension, eine Milch, eine Paste, ein Gel, ein Schaum oder ein Spray, oder als Substrat, das mit dem Mittel imprägniert, getränkt oder beschichtet wurde, vorliegt.

7. Mittel nach einem der Ansprüche 1 bis 6, **dadurch gekennzeichnet, dass** es mindestens einen antibakteriellen Wirkstoff ausgewählt aus Alkoholen, Phenolen, halogenierten Phenolen, etherischen Ölen, PHB-estern, Sorblnsäure, Benzoesäure, Salicylsäure und/oder einem physiologisch verträglichen Salz oder Ester dieser Säuren, Undecensäurederivaten, Formaldehyd, anorganischen Sulfiten und Bisulfiten, 4-Hydroxybenzoesäure und/oder deren physlologisch vertraglichen Salzen oder Estern, Chlorbutanol, Dehydracetsäure, Bronidox, Bronopol, Triclosan, Triclocarben, Chlorcresol, Chlorxylanol, Chlorhexidin, Garmall, Dowicil, Baypival und/oder Kathon enthält.

8. Mittel nach einem der Ansprüche 1 bis 7, **dadurch gekennzeichnet, dass** das Gewichtsverhältnis zwischen dem lichtaktiven Metalloxid und dem antibakteriellen Wirkstoff im Bereich von 200:1 bis 1:10, bevorzugt im Bereich von 100:1 bis 1:5 und insbesondere im Bereich von 50:1 bis 1:1 liegt.

9. Mittel nach einem der Ansprüche 1 bis 8, **dadurch gekennzeichnet, dass** es ein Verdickungssystem enthält, das die Sedimenlaion der Metalloxid-partikel verhindert.

10. Kosmetische Verwendung eines Mittels gemäß einem der Ansprüche 1 bis 9 zur Prophylaxe, Verminderung, Beseitigung und/oder Linderung von bakteriellen, fungiziden und/oder viralen Infektionen.

## Claims

1. Antiseptic cosmetic skin-cleaning agent and/or skin-caring agent, **characterised in that** it comprises a light-active active substance based on a carbon-modified metal oxide that has a specific BET surface area of 200 to 350 m²/g_{.}

2. Agent according to claim 1, **characterised in that** the carbon content of the carbon-modified metal oxide is in the range of 0.01 to 10 wt %, preferably in the range of 0.05 to 5 wt %, particularly preferably in the range of 0.3 to 1.5 wt % and in particular in the range of 0.4 to 0.8 wt %.

3. Agent according to one of claims 1 or 2, **characterised in that** the particle size of the modified metal oxide is in the range of 2 to 600 nm and preferably in the range of 200 to 400 nm.

4. Agent according to one of claims 1 to 3, **characterised in that** it comprises 0.01 to 20 wt %, preferably 0.05 to 10 wt % and in particular 0.1 to 5 wt % of the modified metal oxide, based on the total agent.

5. Agent according to one of claims 1 to 4, **characterised in that** the metal oxide is selected from zinc oxide and/or titanium oxide, in particular titanium oxide.

6. Agent according to one of claims 1 to 5, **characterised in that** it is present as a rinse-off product such as a hand washing soap, a liquid hand washing agent, a soap, a syndet, a shower gel or a shower bath, or as a leave-on product such as an aqueous, alcoholic or aqueous/alcoholic dispersion, an oil dispersion, a balm, a cream, an emulsion, a suspension, a milk, a paste, a gel, a foam or a spray, or as a substrate that was impregnated, soaked or coated with the agent.

7. Agent according to one of claims 1 to 6, **characterised in that** it comprises at least one antibacterial active substance selected from alcohols, phenols, halogenated phenols, ethereal oils, PHB esters, sorbic acid, benzoic acid, salicylic acid and/or a physiologically acceptable salt or ester of these acids, undecenoic acid derivatives, formaldehyde, inorganic sulfites and bisulfites, 4-hydroxybenzoic acid and/or its physiologically acceptable salts or esters, chlorobutanol, dehydracetic acid, Bronidox, Bronopol, Triclosan, Triclocarben, chlorocresol, chloroxylenol, Chlorhexidin, Germall, Dowicil, Baypival and/or Kathon.

8. Agent according to one of claims 1 to 7, **characterised in that** the weight ratio between the light-active metal oxide and the antibacterial active substance is in the range of 200:1 to 1:10, preferably in the range of 100:1 to 1:5 and in particular in the range of 50:1 to 1:1.

9. Agent according to one of claims 1 to 8, **characterised in that** it comprises a thickening system that impedes the sedimentation of the metal oxide particles.

10. Cosmetic use of an agent according to one of claims 1 to 9 for the prophylaxis, reduction, elimination and/or alleviation of bacterial, fungicidal and/or viral infections.

## Revendications

1. Agent cosmétique antiseptique pour les soins et/ou le nettoyage de la peau, **caractérisé en ce qu'**il contient une substance active qui s'active sous l'influence de la lumière à base d'un oxyde métallique modifié avec du carbone, qui présente une surface spécifique selon BET de 200 à 350 m²/g.

2. Agent selon la revendication 1, **caractérisé en ce que** la teneur en carbone de l'oxyde métallique modifié avec du carbone se situe dans la plage de 0,01 à 10 % en poids, de préférence dans la plage de 0,05 à 5 % en poids, de manière particulièrement préférée dans la plage de 0,3 à 1,5 % en poids, et en particulier dans la plage de 0,4 à 0,8 % en poids.

3. Agent selon l'une quelconque des revendications 1 ou 2, **caractérisé en ce que** la granulométrie de l'oxyde métallique modifié se situe dans la plage de 2 à 600 nm et de préférence dans la plage de 200 à 400 nm.

4. Agent selon l'une quelconque des revendications 1 à 3, **caractérisé en ce qu'**il contient - rapportés à l'agent dans sa totalité - de 0,01 à 20 % en poids, de préférence de 0,05 à 10 % en poids, et en particulier de 0,1 à 5 % en poids de l'oxyde métallique modifié.

5. Agent selon l'une quelconque des revendications 1 à 4, **caractérisé en ce que** l'oxyde métallique est choisi parmi l'oxyde de zinc et/ou l'oxyde de titane, en particulier l'oxyde de titane.

6. Agent selon l'une quelconque des revendications 1 à 5, **caractérisé en ce qu'**il est présent sous la forme d'un produit « rinse-off » tel qu'un savon de lavage pour les mains, un agent de nettoyage liquide pour les mains, un savon, un détergent synthétique, un gel pour la douche ou un gel pour le bain, ou sous la forme d'un produit « leave-on » tel qu'une dispersion aqueuse, alcoolique ou aqueuse/alcoolique, une dispersion huileuse, une pommade, une crème, une émulsion, une suspension, un lait, une pâte, un gel, une mousse ou un spray, ou sous la forme d'un substrat qui a été imprégné, imbibé ou enduit avec l'agent.

7. Agent selon l'une quelconque des revendications 1 à 6, **caractérisé en ce qu'**il contient au moins une substance active antibactérienne choisie parmi des alcools, des phénols, des phénols halogénés, des huiles éthérées, des esters PHB, l'acide sorbique, l'acide benzoïque, l'acide salicylique et/ou un sel ou un ester physiologiquement acceptable de ces acides, des dérivés de l'acide undécéneoïque, le formaldéhyde, des sulfites et des bisulfites inorganiques, l'acide 4-hydroxybenzoïque et/ou ses sels ou ses esters physiologiquement acceptables, le chlorobutanol, l'acide déshydroacétique, le bronidox, le bronopol, le triclosan, le triclocarbène, le chlorocrésol, le chloroxylénol, la chlorhexidine, le germall, le dowicil, le baypival et/ou le kathon.

8. Agent selon l'une quelconque des revendications 1 à 7, **caractérisé en ce que** le rapport pondéral entre l'oxyde métallique qui s'active sous l'influence de la lumière et la substance active antibactérienne se situe dans la plage de 200:1 à 1:10, de préférence dans la plage de 100:1 à 1:5, et en particulier dans la plage de 50:1 à 1:1.

9. Agent selon l'une quelconque des revendications 1 à 8, **caractérisé en ce qu'**il contient un système épaississant qui empêche la sédimentation des particules d'oxyde métallique.

10. Utilisation cosmétique d'un agent selon l'une quelconque des revendications 1 à 9 pour la prophylaxie, la réduction, la suppression et/ou le soulagement d'infections bactériennes, fongicides et/ou Virales.
